# FASCICULE DE BREVET EUROPEEN

(11) **EP 2 811 978 B1**
(45) Date de publication et mention de la délivrance du brevet: **20.12.2017**
(21) Numéro de dépôt: 13706653.6
(22) Date de dépôt: 07.02.2013
(51) Int. Cl.: A61K 8/97, A61Q 19/08

(54) **UTILISATION D'UN EXTRAIT D'ECORCE DE POMMIER DANS UNE COMPOSITION COSMETIQUE ANTI-AGE**
VERWENDUNG EINES EXTRAKTES AUS DER APFELBAUMRINDE ZUR KOSMETISCHEN VERHINDERUNG VON ALTERSERSCHEINUNGNEN DER HAUT
USE OF AN APPLE TREE BARK EXTRACT IN A COSMETIC ANTI-AGEING COMPOSITION

(30) Priorité: 09.02.2012 FR 1251226
(43) Date de publication de la demande: 17.12.2014
(73) Titulaire: SOCIETE DE RECHERCHE COSMETIQUE SARL, 2314 Luxembourg (LU)
(72) Inventeur: LECONTE, Nadine, F-92600 Asnieres sur Seine (FR); LECLERE, Jacques, F-45500 Saint-Gondon (FR)
(74) Mandataire: Goulard, Sophie
(86) Numéro de dépôt international: PCT/FR2013/050262
(87) Numéro de publication internationale: WO 2013/117867

(56) Documents cités:
- EP-A2- 1 837 056
- WO-A1-2007/026101
- FR-A1- 2 799 121
- FR-A1- 2 905 854
- Phyto Nutraceutical Inc.: "Apple Tree bark extract", China (Mainland) , 14 novembre 2011 (2011-11-14), XP002689189, Extrait de l'Internet: URL:http://abotanic-pigment.en.gongchang.c om/product/13962587 [extrait le 2012-12-13]
- JP ORTONNE ET AL: "Cellulite and skin ageing: is there any interaction?", JOURNAL OF THE EUROPEAN ACADEMY OF DERMATOLOGY AND VENEREOLOGY, vol. 22, no. 7, juillet 2008 (2008-07), pages 827-834, XP055100619, ISSN: 0926-9959, DOI: 10.1111/j.1468-3083.2007.02570.x
- SY JYE LEU ET AL: "Phenolic Constituents of Malus doumeri var. formosana in the Field of Skin care", BIOL. PHARM. BULL., vol. 29, no. 4, 1 April 2006 (2006-04-01), pages 740-745, XP055325056,
- XU K ET AL: "High-speed counter-current chromatography preparative separation and purification of phloretin from apple tree bark", SEPARATION AND PURIFICATION TECHNOLOGY, ELSEVIER SCIENCE, AMSTERDAM, NL, vol. 72, no. 3, 11 May 2010 (2010-05-11), pages 406-409, XP027019723, ISSN: 1383-5866 [retrieved on 2010-02-26]

## Description

La présente invention concerne une nouvelle composition à base d'extrait d'écorce de pommier, utilisable en cosmétique, plus particulièrement une composition comprenant un extrait d'écorce de pommier utile pour lutter contre les signes du vieillissement cutané, tel que décrit dans les revendications. La peau comprend des couches superficielles, à savoir l'épiderme, et des couches plus profondes, le derme et l'hypoderme, et chacune possède des propriétés spécifiques permettant à l'ensemble de réagir et s'adapter aux conditions de son environnement. L'épiderme, qui est composé de trois types de cellules, à savoir des kératinocytes (90% des cellules épidermiques), des mélanocytes (2 à 3% des cellules épidermiques) et des cellules de Langerhans, constitue la couche externe et joue un rôle fondamental pour assurer la protection et le maintien d'une bonne trophicité. Le derme sert de support à l'épiderme et est principalement constitué de fibroblastes et d'une matrice extracellulaire essentiellement à base de collagène et d'élastine. Les fibres de collagène contribuent à la texture et la tonicité de la peau et l'élastine est responsable de son élasticité. D'autres cellules, comme les macrophages et les leucocytes, sont également présentes dans la couche du derme. L'hypoderme, qui est la couche la plus profonde de la peau, contient les adipocytes qui produisent des lipides pour que le tissu sous-cutané fabrique une couche grasse protégeant les muscles, les os et les organes internes contre les chocs.

Le vieillissement de la peau est un processus qui peut être intrinsèque et naturel, ou extrinsèque c'est-à-dire provoqué par l'environnement, y compris l'exposition au soleil, les variations de températures et les radicaux libres. Le vieillissement de la peau provoque notamment une diminution des synthèses protéiques (collagène, élastine), une diminution de la synthèse des protéo-glycanes, ainsi qu'une accumulation des métalloprotéinases de type MMP3. Les premiers signes du vieillissement de la peau, tels que les rides et ridules, peuvent s'accompagner de marques pigmentaires, d'une diminution de l'épaisseur. Ce vieillissement de la peau se traduit notamment par une perte de fermeté, une modification de l'ovale du visage, une moindre élasticité, une diminution de l'hydratation.

Dans une peau normale, la production endogène de radicaux libres, c'est-à-dire la lipoperoxydation physiologique, est contrebalancée par les mécanismes de défense de l'organisme. Toutefois, de nombreuses circonstances peuvent entraîner un excès de radicaux libres, c'est-à-dire une lipoperoxydation induite, et ces radicaux libres en excès, en agissant sur les phospholipides membranaires, perturbent les propriétés physico-chimiques de la membrane cellulaire et provoquent la formation de dérivés lipidiques oxygénés cytotoxiques et de produits de dégradation tels que le malondialdéhyde qui réagissent avec les protéines. Toutes ces réactions peuvent dénaturer les protéines, altérer les fonctions enzymatiques et dénaturer ou modifier l'ADN cellulaire.

De plus, l'efficacité des systèmes de protection de l'organisme contre les effets des radicaux libres a tendance à diminuer avec l'âge des individus, et il est donc souhaitable de pouvoir disposer de produits renforçant la résistance aux effets des radicaux libres.

Diverses compositions à base de substances d'origine végétale ont été proposées pour traiter les symptômes du vieillissement de la peau et en particulier pour éviter ou neutraliser les radicaux libres. Le brevet FR 2.761.607 décrit une composition comportant un dérivé de silanol méthylé et un dérivé d'une protéine végétale hydrolysée, additionnée le cas échéant d'un dérivé de vitamine C et de vitamine E pour inhiber la production de radicaux libres. Le brevet FR 2.810.424 décrit une composition à base de polyphénols de cacao présentant de bonnes propriétés antiradicalaires. Une composition à base d'extrait de graines de mimosa, permettant d'agir contre l'apparition des rides et la perte d'élasticité de la peau, est décrite dans la demande WO 2007144518.

A cet effet, un grand nombre d'extraits de plantes ont été proposés comme matières de compositions cosmétiques, y compris des extraits d'arbres fruitiers, par exemple l'écorce, les feuilles et les fruits, en fonction des effets recherchés, notamment de diverses plantes de la famille des Rosaceae, telles que poiriers et pommiers.

Le pommier (Pyrus malus) est un des arbres fruitiers les plus cultivés dans le monde, en Chine, en Amérique du Nord et en Europe, dans des régions généralement tempérées, produisant de nombreuses variétés de fruits.

Le fruit du pommier a été utilisé depuis de nombreuses années en cosmétique, et par exemple diverses compositions cosmétiques contenant des extraits de pomme ont été proposées, comme des extraits de fruit de Pyrus malus qui sont utilisés dans des shampooings et dans des crèmes de soins cosmétiques. La pomme contient aussi des pectines qui peuvent être utiles en raison de leur effet hypocholestérolémiant.

Ainsi, le brevet FR 2.719.473 décrit une composition cosmétique comprenant un extrait sec de jus de fruit de Rosaceae tel que poire, pomme, prune, abricot, etc, riche en acide malique, en acide citrique et en sorbitol présentant un effet antiradicalaire et un effet favorisant le renouvellement cellulaire. La demande WO 01 /24806 décrit des extraits secs de branches de pommier susceptibles de procurer des effets amincissants. Le brevet EP 1.338.270 décrit l'utilisation de fractions phénoliques d'extraits de fruits de la famille des Rosaceae, notamment la pomme et plus particulièrement l'espèce Malus sylvestris, dont la fraction polyphénolique est riche en dihydrochalcones permettant la préparation de compositions utiles en cosmétique et en nutraceutique pour limiter la surcharge pondérale en raison de l'effet de diminution de l'absorption des sucres par les tissus cutanés résultant principalement de la présence de phloridzine dans les extraits. On sait que la phloridzine extraite de l'écorce de pommier Malus pumila Mill est un actif utile en thérapeutique pour traiter diverses affections, comme indiqué dans la note du fabricant Phyto Nutraceutical Inc sur son site abotanic-pigment.en.gongchang.com/product/13962587. La pomme contient aussi des pectines qui peuvent être utiles en raison de leur effet hypocholestérolémiant, ainsi que des polyphénols à action anti-oxydante.

De surcroît, la demande FR 2.799.121 décrit des extraits de branches de pommiers comprenant dans l'extrait sec 10 à 25% de phloridzine et 20-40% de polyphénols pour une utilisation anti-cellulite. Sy Jye Leu et al. (2006, Biol. Pharm. Bull. 29(4) 740-745) font une analyse d'extraits de feuilles de pommiers taiwanais et proposent d'utiliser certaines des molécules aux propriétés anti-radicaux et anti-élastase comme agents anit-vieillissement de la peau. Enfin la demande WO 2007/026101 A1 propose des extraits issus des fruits, peaux et pépins de pommes à des fins d'actifs anti-age pour la peau. Par contre, les études réalisées par la demanderesse sur des fibroblastes humains en culture et sur des épidermes reconstitués ont montré de manière inattendue qu'un extrait spécifique d'écorce de pommier possède une activité antiradicalaire et anti-élastase utile pour la préparation de compositions cosmétiques destinées à protéger la peau et lutter contre les effets du vieillissement cutané.

Selon la présente invention l'extrait spécifique d'écorce de pommier est un extrait aqueux d'écorce de pommier obtenu par macération dans l'eau à partir d'écorces préalablement séchées et broyées, ledit extrait comprenant entre 5 et 10 % de tanins (matières sèches) et entre 2 et 10 % de phloridzine (matière sèche) ayant un effet anti-radicalaire et anti-élastase pour la prévention et le traitement des effets du vieillissement cutané. La présente invention a donc pour objet l'utilisation d'extraits d'écorce de pommier selon l'invention pour la préparation d'une composition cosmétique destinée à la prévention et au traitement des effets du vieillissement cutané.

L'invention a encore pour objet un procédé de traitement cosmétique non thérapeutique de la peau par application d'une composition à base d'extrait d'écorce de pommier selon l'invention sur la zone de la peau nécessitant un tel traitement, notamment la face et le cou.

Plus particulièrement, il a été démontré qu'un extrait d'écorce de pommier selon l'invention
- présente une parfaite innocuité vis-à-vis des fibroblastes humains en culture ;
- inhibe l'activité de l'élastase au niveau des fibroblastes humains en culture.
- présente un excellent effet antiradicalaire.

Une propriété remarquable des extraits d'écorce de pommier de l'invention est l'obtention d'un effet antiradicalaire très net, même avec une faible quantité de matière sèche. L'activité anti-élastase permet de compléter cet effet antiradicalaire sur le plan de l'efficacité cosmétique.

Les extraits d'écorce de pommier suivant l'invention peuvent être obtenus à partir de pommiers de la famille des Rosaceae, de préférence des espèces Pyrus malus ou Malus sylvestris Mill., Malus domestica, Malus pumila, Malus floribunda, Malus coronaria, etc, dont on connaît un grand nombre de variétés.

L'écorce peut être prélevée sur des branches issues de la taille des pommiers. Les extraits utilisés dans la présente invention sont obtenus à partir d'écorce des branches de pommier, de préférence par séchage et broyage des écorces, suivi d'une macération ou d'une extraction par un solvant et séparation des phases liquides et solides. Le solvant d'extraction éventuellement utilisé peut être l'eau, ou un alcool tel que le 1,3-propanediol ou le 1,3-butanediol, ou un mélange eau/alcool. La méthode d'extraction préférée est la macération à une température de 20 à 40°C. Suivant une variante on peut procéder par percolation au moyen d'une poudre de granulométrie appropriée.

L'extrait d'écorce de pommier utilisé dans les compositions suivant la présente invention est de préférence sous forme d'extraits aqueux de l'écorce séchée et réduite en poudre par broyage, obtenus par macération dans l'eau à raison d'environ 80 à 120 g/l pendant deux à trois jours sous agitation à température ambiante. Après décantation, le gâteau est exprimé et les liqueurs obtenues sont jointes avant d'être concentrées le cas échéant.

Suivant une forme avantageuse de réalisation, l'extrait utilisable dans la composition de l'invention est stabilisé au moyen d'un conservateur approprié tel qu'alcool benzylique et acide déhydroacétique, permettant d'éviter toute action enzymatique ou oxydante susceptible de dénaturer le produit.

L'extrait d'écorce de pommier utilisé dans la présente invention est un extrait aqueux obtenu à partir de pommiers biologiques de Provence (Pyrus malus) et se présente sous la forme d'un liquide de couleur jaune pâle à ambré, d'odeur caractéristique, soluble à 10% dans l'eau et dans l'éthanol, se caractérisant par :
- pH 4,6 - 6,6
- matières sèches ≥ 1,0 %
- indice de réfraction à 22°C 1,330 - 1,380
- densité à 20°C 0,950 - 1,050
- tanins (matières sèches) 5 - 10%
- phloridzine (matières sèches) 2 - 10%

Dans la composition selon l'invention, la teneur en extrait d'écorce de pommier peut être comprise entre 1,0 et 20%, et de préférence entre 2 et 15% en poids par rapport au poids total de la composition, pour procurer les meilleurs effets cosmétiques.

L'extrait d'écorce de pommier peut être utilisé avantageusement sous une forme encapsulée dans des liposomes.

Suivant une technique connue dans la fabrication des compositions cosmétiques, les liposomes sont constitués par des petites sphères creuses, de diamètre généralement inférieur à 500 nm, dont la paroi est formée d'une double couche de lipides tels que des glucolipides ou des phospholipides. Ils peuvent être obtenus par exemple par traitement aux ultrasons d'un mélange d'un soluté aqueux et de lipides. Les lipides (phospholipides ou glucolipides) se réorganisent dans une configuration où l'énergie de l'ensemble est minimale, donc thermo-dynamiquement la plus stable. Les liposomes sont utilisés dans l'industrie cosmétique pour délivrer des composés à l'intérieur des cellules lorsque le vésicule fusionne avec la membrane plasmique.

Suivant une forme de réalisation de l'invention, une partie au moins des extraits est encapsulée dans des vésicules du type liposome.

Les compositions conformes à la présente invention sont de préférence administrables par voie topique. Suivant la terminologie classique, l'administration par voie topique désigne toute méthode consistant à appliquer la substance ou la composition directement sur la peau, sur la zone nécessitant le traitement.

Conformément à la présente invention, la composition administrable par voie topique peut avantageusement contenir, outre les composants de base décrits ci-dessus, une ou plusieurs autres substances connues pour exercer des effets complémentaires bénéfiques pour la peau, et plus particulièrement le tocophérol, la vitamine A (rétinol), l'acide rétinoïque, le bisabolol, des agents bactéricides, du monométhylsilanol, de la proline, ou encore du beurre de Karité.

On peut aussi ajouter à la composition des extraits ou composés connus pour faciliter la pénétration des principes actifs de la composition à travers l'épiderme, par exemple des saponosides du type hédéragénine.

Les compositions cosmétiques et pharmaceutiques conformes à la présente invention, sont destinées de préférence à une administration topique, et contiennent donc des supports et excipients couramment utilisés dans des compositions de ce type telles que des émulsions H/E ou E/H, des crèmes, des gels ou des lotions. Dans le cas des émulsions, la phase grasse peut représenter entre 10 et 60% environ du poids de la composition, la phase aqueuse entre 10 et 80% environ et l'agent émulsionnant entre 2 et 20%, le reste étant constitué par les composants de base indiqués ci-dessus et les autres composants mentionnés ci-après.

La composition peut encore contenir diverses substances et excipients choisis en fonction de leurs propriétés connues et de la forme galénique envisagée. Ainsi, on peut incorporer dans la composition des conservateurs, des agents émulsionnants, des agents viscosants, des épaississants, des gélifiants, des agents de chélation, des antioxydants, des agents hydratants, des tensioactifs, des parfums, des huiles, des lipides, un solvant spécifique ainsi que de l'eau et divers additifs destinés à améliorer les propriétés physiques de la composition.

On peut choisir l'agent émulsionnant parmi des polymères carboxyvinyliques à haut poids moléculaire, des polysorbates (par exemple le Tween 20® et le Tween 60®), des esters de sorbitan et en particulier un monostéarate tel que le Span 60®, un tristéarate, un monopalmitate et un laurate de sorbitan. On peut encore utiliser d'autres agents émulsionnants tels que divers dérivés d'acide stéarique ou palmitique, et par exemple le stéarate de PEG 100®, des mono- ou diglycérides d'acide stéarique ou palmitique, un ester de polyglycérol, un ester gras d'acide citrique ou tartrique, un stéarate de propylène glycol auto-émulsionnable, ou encore le polyglycéryl-2-sesquioléate, l'éther cétylique de polyoxy-éthylène, un polyglucoside de siloxane, ou une silicone émulsionnable. On peut aussi utiliser un émulsionnant choisi parmi les lécithines hydrogénées, les stérols végétaux (par exemple de soja), des lipoprotéines et des sphingolipides.

Les agents viscosants utilisés dans les compositions de l'invention peuvent être choisis parmi divers polymères d'acide acrylique, un copolymère d'acrylate et de laurate d'acryloyle, une gomme cellulose, une gomme arabique, le Sclerotium gum, le pullulan, un mucilage de Gleditsia, une silice, des polymères carboxyvinyliques, un silicate d'aluminium et de magnésium, et on peut utiliser par exemple la silice colloïdale vendue sous la marque Aerosil 200® ou un acide polyacrylique réticulé tel que le Carbopol 940®.

Les gélifiants ou épaississants peuvent être choisis par exemple parmi les polyacrylamides, des acrylates comme le Pemulen®, les dérivés de cellulose comme l'hydroxypropyl cellulose, ou les gommes naturelles telles qu'une gomme Xanthane.

Les agents chélatants permettent d'améliorer la stabilité des produits dans le temps et peuvent être choisis par exemple parmi l'EDTA disodique ou tétrasodique, l'acide oxalique ou l'acide galactarique, des dérivés de l'acide panthétique ou de l'acide étidronique, ainsi que l'acide phytique.

Les agents hydratants utilisés peuvent être choisis par exemple parmi un polyol, le sorbitol, le maltitol, le xylitol, le pentaérythritol, le butylène glycol, l'alcool D-panthotényl (Panthenol), les polyacrylates et polyméthacrylates de glycéryle, le glycérol ou des dérivés de glycérol, le sel de sodium de l'acide pyrrolidone carboxylique (sodium PCA), ou encore une argile hydratante comme le Veegum HV (silicate de magnésium / aluminium) qui a aussi pour effet de stabiliser l'émulsion. On peut encore ajouter des émollients tels qu'un malate d'alkyle, l'isohexadécane, des triglycérides d'acide caprique ou caprylique, l'alcool béhénylique, etc.

Les conservateurs usuels de la technique des compositions dermatologiques ou cosmétologiques peuvent être utilisés dans l'invention, et par exemple l'acide déhydroacétique et son sel de sodium, l'acide benzoïque et un p-hydroxybenzoate d'alkyle tel que le p-hydroxy-benzoate de méthyle (Méthylparaben), un alcool tel que le phénoxy-éthanol, l'alcool benzylique ou encore la chlorphénésine ou l'imidazolidinyl urée, ainsi que des dérivés d'acide vanillique comme l'acide anisique, l'acide lévulinique, l'acide gluconique et le gluconate de calcium.

Les constituants de la phase grasse, c'est-à-dire les huiles et lipides, peuvent être choisis parmi l'huile de jojoba, l'huile de maïs, l'huile de vaseline, l'huile d'amandes douces, l'huile de coco hydrogénée, l'huile de carthame, des glycérides d'acides gras saturés, l'acide stéarique, l'acide palmitique, le stéarate d'octyle, le palmitate de glycéryle, le palmitate d'octyle, un triglycéride d'acides caprique et caprylique, le 2-octyl-dodécanol, le polyéthylène glycol, l'adipate d'éthyl-2 hexyle, ou encore des huiles de silicones telles que le méthyl phényl polysiloxane, la diméthicone, la cyclométhicone et la phényl diméthicone.

La composition peut aussi contenir un solvant choisi en fonction des composants utilisés et de la forme d'administration envisagée. Le solvant peut être par exemple de l'eau, et de préférence de l'eau déminéralisée, ou un solvant spécifique tel que le propylène glycol, le butylène glycol-1,3, le butylène glycol-1,4, un éther de diéthylène glycol, ou un alcool tel que l'éthanol.

Des agents de protection contre les rayons ultraviolets peuvent aussi être avantageusement incorporés dans les compositions, et par exemple des filtres solaires UV-A et UV-B hydrophiles ou lipophiles. On peut aussi utiliser des pigments formant écran anti-ultraviolet, comme par exemple le dioxyde de titane, l'oxyde de zinc et l'oxyde de zirconium.

Le pH de la composition est de préférence compris entre 5,3 et 7,5, et peut être ajusté, selon les compositions, par addition d'un acide tel que l'acide citrique ou d'une base telle que l'hydroxyde de sodium ou de potassium.

La composition conforme à la présente invention peut être présentée sous les formes classiquement utilisées pour une application topique, c'est-à-dire sous forme de gel, lotion, émulsion (en particulier crème ou lait), patchs transdermiques, masque ou pommade, contenant des excipients et supports usuels compatibles et physiologiquement acceptables. Ces formes d'administration par voie topique sont préparées par les techniques connues, et par exemple, dans le cas d'une crème, par dispersion d'une phase grasse dans une phase aqueuse pour obtenir une émulsion huile dans eau, ou inversement pour préparer une émulsion eau dans huile. Dans le cas de crèmes, on préfère utiliser des émulsions à structure lamellaire contenant peu de produits éthoxylés ou n'en contenant pas du tout. L'extrait d'écorce de pommier utilisé dans l'invention est introduit dans la phase aqueuse ou alcoolique.

A titre d'exemple, on peut préparer des compositions topiques conformes à l'invention sous forme de crèmes, de lotions ou de gels, utilisables en une ou plusieurs applications quotidiennes, la durée du traitement pouvant être généralement de l'ordre de un à plusieurs mois, les compositions pouvant être utilisées sans inconvénient de manière continuelle.

Les exemples de compositions donnés ci-après illustrent d'avantage l'invention. Sauf indication contraire, les parties et pourcentages sont indiqués en poids.

### Exemple 1

Une crème protectrice à base d'extrait d'écorce de pommier biologique de Provence ayant la composition indiquée ci-après est préparée suivant les techniques usuelles :

| Phase A | | |
|---|---|---|
| Eau déminéralisée | q.s.p. | 100,00 |
| EDTA disodique | | 0,10 |
| Déhydroacétate de sodium | | 0,15 |
| Alcool benzylique | | 0,30 |
| Acide lévulinique | | 0,30 |
| Glycérine | | 4,50 |

| Phase B | | |
|---|---|---|
| Polymère carboxyvinylique | | 0,15 |

| Phase C | | |
|---|---|---|
| Tween 60 | | 7,50 |
| Span 60 | | 2,50 |
| Cire d'abeille | | 2,50 |
| Alcool béhénylique | | 2,00 |
| Huile de tournesol | | 3,00 |
| Beurre de Karité | | 1,50 |
| Perhydroxysqualène végétale | | 2,50 |
| Triglycérides caprique / caprylique | | 4,50 |
| Acide stéarique | | 1,00 |
| Glycéryl undécylate | | 0,30 |
| Tocophérol | | 0,70 |

| Phase D | | |
|---|---|---|
| Panthenol | | 0,50 |
| Sodium PCA | | 1,00 |

| Phase E | | |
|---|---|---|
| Bisabolol | | 0,20 |

| Phase F | | |
|---|---|---|
| Extrait d'écorce de pommier | | 5,00 |
| Eau de fleur de souci | | 1,50 |

| Phase G | | |
|---|---|---|
| Silicium (Methyl polysiloxane V100) | | 0,50 |
| Parfum | | 0,50 |

La phase aqueuse A est chauffée à 80°C pour faire fondre les composants, puis on ajoute la phase B pour former un gel, tandis que la phase grasse B est chauffée à environ 80°C puis mélangée soigneusement sous agitation avec le gel. Les phases D à F sont ajoutées successivement à 50°C environ. L'ensemble est mélangé et refroidi progressivement et le parfum est ajouté à environ 40°C.

Cette crème protectrice peut être utilisée par application sur les zones de la peau à traiter, une à deux fois par jour, pendant plusieurs semaines.

### Exemple 2

Un sérum à base d'extrait d'écorce de pommier biologique de Provence ayant la composition indiquée ci-après est préparé suivant les techniques usuelles :

| Phase A | | |
|---|---|---|
| Eau déminéralisée | q.s.p. | 100,00 |
| Glycerine | | 4,00 |
| Sclerotium gum | | 0,50 |
| Gomme Xanthane | | 0,50 |

| Phase B | | |
|---|---|---|
| Alcool éthylique 96% vol | | 4,00 |
| Bicaprylyl ether | | 1,00 |
| Déhydroacétate de sodium | | 0,15 |
| Acide lévulinique | | 0,30 |
| Alcool benzylique | | 0,30 |
| Acide galactarique | | 0,10 |
| Tocophérol | | 0,10 |
| Isostéaryl isononanoate | | 0,80 |

| Phase C | | |
|---|---|---|
| Aspartate de lysine | | 1,00 |
| Acide lactique (and) eau (and) arginine | | 5,00 |
| Extrait d'écorce de pommier | | 10,00 |

Les composants de la phase A sont mélangés à chaud de manière usuelle pour former un gel, puis on ajoute successivement les phases B et C pour former un sérum qui peut être utilisé pour les soins, en particulier, du visage et du cou.

### Exemple 3

### Evaluation de la cytotoxicité

La cytotoxicité des extraits d'écorce de pommier suivant l'invention a été étudiée vis-à-vis de fibroblastes humains en culture.

Des cultures de fibroblastes ont été établies à partir de prélèvements de peau humaine et ont été amplifiées en milieu de culture cellulaire RPMI 1640 supplémenté par du sérum de veau foetal, de la L-glutamine et de la pénicilline/streptomycine. Les essais sont effectués sur des fibroblastes entre le 4^{ème} et le 6^{ème} passage. Les fibroblastes sont ensemencés sur des plaques 6 puits à raison de 10⁵ cellules par ml. Ils sont ensuite incubés pendant 24 heures.

Deux lots ont été constitués :
Lot n°1 : témoin ne recevant aucun produit.
Lot n°2 : traité avec l'extrait aqueux d'écorce de pommier à 1,0 %.

Chaque lot a été maintenu au contact des fibroblastes humains en culture pendant 24 heures.

La viabilité cellulaire est déterminée par le test de réduction au bleu de Formazan (test MTT) après 24 heures d'incubation des cellules de fibroblastes.

Après incubation des cellules avec chacun des lots de produit à l'étude, les puits contenant les cellules sont vidés par retournement doux et le tapis cellulaire est rincé avec le milieu de culture. On distribue dans tous les puits 200 µl d'une solution de MTT (bromure de 3-(4,5-diméthylthiazol-2-yl)-2,5-diphényltétrazolium) diluée au moment de l'emploi (5 mg/ml), puis les plaques sont incubées à 37°C pendant 3 heures.

Les puits sont à nouveau vidés par retournement doux et remplacés pendant 1 minutes par 200 µl d'une solution de formo-calcium pour fixer le tapis cellulaire. On observe alors la formation de cristaux de bleu de Formazan. Les cellules sont ensuite lysées et les cristaux de bleu de Formazan sont dissous par addition de 200 µl de diméthylsulfoxyde (DMSO). Après homogénéisation de la coloration par agitation, la densité optique (DO) des plaques est lue, au moyen d'un spectrophotomètre à 570 nm, ce qui permet de connaître la quantité relative de cellules vivantes et actives métaboliquement.

Les valeurs de la densité optique (DO) après 24 heures de contact sont regroupées dans le tableau ci-dessous.

| Substance | Densité optique DO 570 nm | % |
|---|---|---|
| Lot n°1 (témoin) | 0,540 ± 0,07 | - |
| Lot n°2 (1,0 %) | 0,552 ± 0,09 | ns |

Ces résultats montrent que l'extrait aqueux d'écorce de pommier à 1,0 % utilisé dans l'invention ne présente aucune cytotoxicité vis-à-vis des fibroblastes humains en culture aux concentrations étudiées.

### Exemple 4

L'évaluation de l'activité anti-élastase des extraits d'écorce de pommier utilisés dans l'invention, au niveau des fibroblastes humains en culture, a été effectuée comme indiqué ci-après.

L'évaluation a été faite sur les mêmes fibroblastes humains en culture que ceux de l'Exemple 3 ci-dessus.

Les lots témoin et extrait à la concentration de 1,0% sont constitués comme suit :
Lot n° 1 : témoin ne recevant aucun produit.
Lot n° 2 : traité avec l'extrait aqueux d'écorce de pommier à 1 % + SANA.

Les essais sont réalisés en triplicate après 24 heures de contact des cellules de fibroblastes avec chaque lot en présence de substrat N-succinyl trialanine paranitroanilide (SANA).

Les essais sont réalisés sur des fibroblastes entre le 4^{ème} et le 6^{ème} passage. Les fibroblastes sont ensemencés sur des plaques multipuits à raison de 10⁵ cellules / puits. Ils sont ensuite incubés avec 2% de SVF et RMPI pendant 24 heures. Le SVF est ensuite remplacé par 0,2% de BSA et les cellules sont incubées pendant 24 heures en présence des produits à l'étude. En fin de traitement, les cellules sont récupérées par grattage et les enzymes sont extraites du culot cellulaire en utilisant 0,1% de Triton X-100 dans un tampon Tris-HCl à pH 8, Brij 35 0,1% et 20 µl d'une solution de N-succinyl trialanine paranitroanilide (SANA, 125 mM) dans de la N-éthylpyrrolidone. La réaction est initiée à 37°C et stoppée par ajout de 50 µL d'acide acétique. La densité optique est mesurée à 410 nm au moyen d'un spectrophotomètre.

Les unités enzymatiques sont définies en nM de nitroaniline libérée par heure et par 10⁵ cellules.

Les résultats sont regroupés dans le tableau ci-dessous.

| Substance | Activité de l'élastase (nM de SANA hydrolysé / heure / 10⁵ cellules) | % |
|---|---|---|
| Lot n°1 (témoin) | 61,7 ± 4,2 | - |
| Lot n°2 (1,0 %) | 55,8 ± 3,2 | - 10 |

Ces résultats montrent que l'extrait d'écorce de pommier utilisé dans l'invention, à la concentration de 1,0 %, inhibe de - 10%, l'activité de l'élastase au niveau des fibroblastes humains en culture.

### Exemple 5

L'activité antiradicalaire a été mise en évidence par une étude sur des épidermes humains reconstitués (Skinethics®) décrits par exemple par M. Rosdy, "Reconstituted epidermis for testing", Cosmetics and Toiletries Manuf. Worldwide, Aston Publ. Group. 223-226 (1994).

Suivant cette technique, des épidermes sont formés à partir de kératinocytes d'origine humaine ensemencés sur des filtres en polycarbonate dans un milieu défini (MCDB 153 modifié) et supplémenté, les cellules étant cultivées pendant 14 jours à l'interface air/liquide.

L'activité antiradicalaire a été évaluée par dosage du malondialdéhyde (MDA) après son induction par l'hypoxanthine / xanthine oxydase. Le dosage du MDA, qui est l'un des marqueurs essentiels de la cytotoxicité par les processus oxydatifs et le stress, permet de mesurer l'activité antiradicalaire.

Cinq lots ont été constitués :
Lot n° 1 : épiderme témoin ne recevant aucun produit.
Lot n° 2 : témoin positif traité par hypoxanthine / xanthine oxydase.
Lot n° 3 : épiderme traité par Vitamine E + hypoxanthine / xanthine oxydase.
Lot n° 4 : épiderme traité avec l'extrait aqueux d'écorce de pommier à 1,0 %
Lot n° 5 : épiderme traité avec l'extrait aqueux d'écorce de pommier à 1,0 % + hypoxanthine / xanthine oxydase.

Après 24 heures de traitement, les homogénats cellulaires sont remis en suspension dans un tampon approprié à pH 8, on laisse incuber 1 heure dans l'obscurité, on ajoute du n-butanol et on centrifuge pour récupérer la phase supérieure afin de doser le MDA par fluorescence après séparation du complexe MDA-TPA par HPLC.

Le dosage des protéines est fait suivant la méthode de Bradford, par spectrophotométrie.

On détermine ainsi, par dosage du MDA, la lipoperoxydation physiologique, et la lipoperoxydation provoquée par l'hypoxanthine / xanthine oxydase. Les résultats sont regroupés dans les tableaux ci-après.

Lipoperoxydation physiologique :

| Substance | MDA (µM/mg protéine) | Diminution du MDA % |
|---|---|---|
| Lot n°1 (témoin) | 713 ± 57 | - |
| Lot n°4 (1,0 %) | 607 ± 40 | - 15 |

Ces résultats montrent que l'extrait de l'invention procure une protection significative vis-à-vis de la lipoperoxydation physiologique puisque la diminution du taux de MDA est de 15%.

Lipoperoxydation provoquée :

| Substance | MDA (µM/mg protéine) | Diminution du MDA % |
|---|---|---|
| Lot n°1 (témoin) | 713 ± 57 | - |
| Lot n°2 (témoin positif) | 1001 ± 85 | + 40 |
| Lot n°3 | 650 ± 48 | - 35 |
| Lot n°5 | 815 ± 70 | - 19 |

Ces résultats montrent une protection significative de l'extrait de l'invention vis-à-vis de la lipoperoxydation provoquée par l'hypoxanthine / xanthine oxydase.

## Revendications

1. Utilisation d'un extrait aqueux d'écorce de pommier obtenu par macération dans l'eau à partir d'écorces préalablement séchées et broyées, ledit extrait comprenant entre 5 et 10 % de tanins (matières sèches) et entre 2 et 10 % de phloridzine (matière sèche) pour la préparation d'une composition cosmétique ayant un effet anti-radicalaire et anti-élastase pour la prévention et le traitement des effets du vieillissement cutané.

2. Utilisation selon la revendication 1, **caractérisée en ce que** l'extrait est obtenu à partir d'écorces de branches de pommiers des espèces Pyrus malus ou Malus sylvestris Mill., Malus domestica, Malus pumila, Malus floribunda, ou Malus coronaria.

3. Utilisation selon l'une quelconque des revendications précédentes, **caractérisée en ce que** ledit extrait a les caractéristiques suivantes :
- pH 4,6 - 6,6
- matières sèches ≥ 1,0 %
- indice de réfraction à 22°C 1,330 - 1,380
- densité à 20°C 0,950 - 1,050

4. Utilisation selon l'une quelconque des revendications précédentes, **caractérisée en ce que** la teneur en extrait d'écorce de pommier est comprise entre 1,0 et 20% en poids par rapport au poids total de la composition.

5. Utilisation selon la revendication 4, **caractérisée en ce que** la teneur en extrait d'écorce de pommier est comprise entre 2 et 15% en poids par rapport au poids total de la composition.

6. Procédé de traitement cosmétique non thérapeutique de la peau, pour prévenir et traiter les effets du vieillissement cutané, par application d'une composition cosmétique à base d'extrait aqueux d'écorce de pommier obtenu par macération dans l'eau à partir d'écorces préalablement séchées et broyées, ledit extrait comprenant de 5 à 10% de tanins (matières sèches) et de 2 à 10 % de phloridzine (matière sèche) ayant un effet anti-radicalaire et anti-élastase, sur la zone de la peau nécessitant un tel traitement.

## Patentansprüche

1. Verwendung eines wässrigen Apfelbaumrindenextrakts, erhalten durch Mazeration in Wasser aus zuvor getrockneten und zerkleinerten Rinden, wobei der Extrakt zwischen 5 und 10% Tannine (Trockenmasse) und zwischen 2 und 10 % Phloridzin (Trockenmasse) umfasst, für die Herstellung einer kosmetischen Zusammensetzung mit einer Antiradikal- und Antielastasewirkung zur Vorbeugung und Behandlung der Wirkungen der Hautalterung.

2. Verwendung nach Anspruch 1, **dadurch gekennzeichnet, dass** der Extrakt aus Rinden von Zweigen von Apfelbäumen der Spezies Pyrus malus oder Malus sylvestris Mill., Malus domestica, Malus pumila, Malus floribunda oder Malus coronaria erhalten ist.

3. Verwendung nach einem der vorangehenden Ansprüche, **dadurch gekennzeichnet, dass** der Extrakt die folgenden Merkmale hat:
- pH 4,6 - 6,6
- Trockenmasse ≥ 1 ,0 %
- Brechungsindex bei 22 °C 1,330 - 1,380
- Dichte bei 20°C 0,950 - 1,050

4. Verwendung nach einem der vorangehenden Ansprüche, **dadurch gekennzeichnet, dass** der Gehalt an Apfelbaumrindenextrakt zwischen 1,0 und 20 Gew.-% inklusive in Bezug zum Gesamtgewicht der Zusammensetzung beträgt.

5. Verwendung nach Anspruch 4, **dadurch gekennzeichnet, dass** der Gehalt an Apfelbaumrindenextrakt zwischen 2 und 15 Gew.-% inklusive in Bezug zum Gesamtgewicht der Zusammensetzung beträgt.

6. Verfahren zur nicht therapeutischen kosmetischen Behandlung der Haut, um den Wirkungen der Hautalterung vorzubeugen und sie zu behandeln, durch Anwenden einer kosmetischen Zusammensetzung auf der Basis von wässrigem Apfelbaumrindenextrakt, erhalten durch Mazeration in Wasser aus zuvor getrockneten und zerkleinerten Rinden, wobei der Extrakt 5 bis 10% Tannine (Trockenmasse) und 2 bis 10 % Phloridzin (Trockenmasse) mit einer Antiradikal- und Antielastasewirkung auf dem Bereich der Haut, der eine derartige Behandlung benötigt, umfasst.

## Claims

1. Use of an aqueous extract of apple tree bark obtained by maceration in water of previously dried and ground bark, said extract comprising between 5 and 10 % by weight of tannin (dry matter) and between 2 and 10 % of phloridzin (dry matter) to prepare a cosmetic composition having anti-radical and anti-elastase effect for the prevention and treatment of the effects of skin ageing.

2. The use according to claim 1, **characterized in that** the extract is obtained from the bark of branches of apple trees of the species *Pyrus malus* or *Malus sylvestris Mill., Malus domestica, Malus pumila, Malus floribunda* or *Malus coronaria,*

3. The use according to any of the preceding claims, **characterized in that** said extract has the following characteristics:
- pH 4.6 - 6.6
- dry matter ≥ 1.0 %
- refractive index at 22°C 1.330 - 1.380
- density at 20° C 0.950 - 1.050.

4. The use according to any of the preceding claims, **characterized in that** the content of apple tree bark extract is between 1.0 and 20 % by weight relative to the total weight of the composition.

5. The use according to claim 4, **characterized in that** the content of apple tree bark extract is between 2 and 15 % by weight relative to the total weight of the composition.

6. Method for non-therapeutic, cosmetic skin treatment to prevent and treat the effects of skin ageing, by applying a cosmetic composition based on an aqueous extract of apple tree bark obtained by maceration in water of previously dried and ground bark, said extract comprising 5 to 10% of tannin (dry matter) and 2 to 10 % of phloridzin (dry matter) having an anti-radical and anti-elastase effect on a skin region necessitating said treatment.
